# EUROPEAN PATENT APPLICATION

(11) **EP 2 420 245 A1**
(43) Date of publication of application: **22.02.2012**
(21) Application number: 10075353.2
(22) Date of filing: 18.08.2010
(51) Int. Cl.: A61K 38/04, A61P 9/00

(54) **Therapeutic use of agonists or antagonists of bradykinin receptor 1 or 2, for modulation collateral blood vessel growth**

(71) Applicant: MAX-DELBRÜCK-CENTRUM FÜR MOLEKULARE MEDIZIN, 13125 Berlin (DE)
(72) Inventor: Hillmeister, Philipp., 10437 Berlin (DE); Buschmann, Ivo., 13465 Berlin (DE); LeNoble, Ferdinand., 10178 Berlin (DE); Gatzke, Nora., 15907 Berlin (DE)
(74) Representative: Boeckh, Tobias

(57) **Abstract**

The invention relates to a method for modulating collateral blood vessel or artery growth (arteriogenesis), whereby collateral growth is either stimulated or inhibited, in addition to substances capable of such modulation and related medicaments. The present method for modulating collateral blood vessel growth functions via the inhibition or stimulation of the bradykinin receptor 1 (B1 R) and/or the bradykinin receptor 2 (B2R) signalling pathways.

## Description

The invention relates to a method for modulating collateral blood vessel or artery growth (arteriogenesis), whereby collateral growth is either stimulated or inhibited, in addition to substances capable of such modulation and related medicaments. The present method for modulating collateral blood vessel growth functions via the inhibition or stimulation of the bradykinin receptor 1 (B1 R) and/or the bradykinin receptor 2 (B2R) signalling pathways.

### BACKGROUND OF THE INVENTION

Arteriogenesis (collateral blood vessel growth) is the most important endogenous mechanism of compensation for an arterial stenosis or occlusion. The driving force is a pressure gradient, for example through a stenosis or an AV-shunt, which initiates an increased blood flow through the collateral arteries in the areas of hypoperfusion. Arteriogenesis is induced by flow, namely by an increased shear stress which act on endothelial cells. This activates the bradykinin receptor signal pathway, through which arteriogenesis is enhanced. Collateral blood vessel growth can be modified (inhibition or stimulation) through modulation of the bradykinin receptor signal pathway. This enables methods involving the modification of this mechanism for the prevention of circulatory disorders, vascular diseases and/or ischemic arterial blockages or occlusions (KHK, stroke, PAVK). Modulation of arteriogenesis via the bradykinin signalling pathway can also be applied to other illnesses or disorders, for example AV-shunt malformation and the MojaMoja disease.

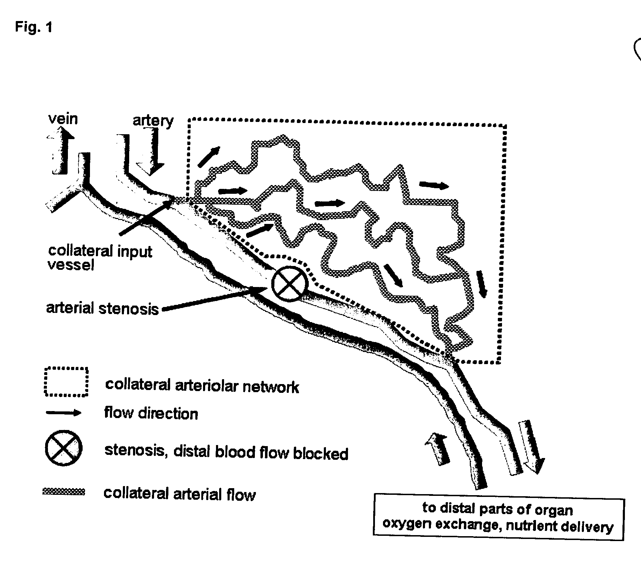

Diseases related to ischemic arterial blockage represent the most common cause of death in the western world (Lloyd-Jones, Adams et al. 2009). The most common causes of stenosis or occlusive diseases of the main arteries of the brain, the heart or the periphery are progressive atherosclerotic modifications of the blood vessels. The possibilities for treating such diseases are at the present time very limited, whereby the therapies on offer are often unsatisfactory (Schiele, Niehues et al. 2000). Angioplasty (balloon dilatations) and bypass operations are the only methods of treatment available in clinics at the present time. An angioplasty can however only be applied in a case of an arterial obstructive or occlusive disease (for example ischemic heart diseases) and as a consequence leads to an increased risk of a restenosis. Bypass surgeries are in any case only applicable for certain arterial obstructive or occlusive diseases and are associated with the disadvantage that an invasive intervention is necessary. New therapeutic strategies are therefore particularly relevant when considering how to treat and to avoid vascular obstructive or occlusive diseases. A pharmaceutical stimulation of arteriogenesis (the concept of growing a biological bypass) represents enormous therapeutic potential and a very promising method in treating or preventing arterial obstructive or occlusive diseases (Love 2003).The present invention therefore relates to the modulation of arteriogenesis and flow-induced collateral blood vessel growth (Fig. 1).

The vascular system is organized in a highly complex manner. In order to ensure the essential functions of distributing oxygen and nutrients, the vascular system is constantly undergoing reconstruction. The reconstruction of blood vessels differs between arteriogenesis on the one hand and angiogenesis on the other hand.

In the context of the present invention it is important to distinguish between arteriogenesis and angiogenesis. Angiogenesis relates to the outgrowth of new capillaries from previously existing vessels. This relates specifically to the growth of new vessels (capillary sprouting). The present invention does not relate to modulation of neo-vascularisation (such as angiogenesis) but rather to modulation of arteriogenesis.

The diameter of an artery is actively increased in arteriogenesis. This mechanism does not deal with a passive dilatation, rather an active reconstruction of the vessel including the degradation of the extracellular matrix and a proliferation of artery cells (endothelial cells and smooth muscle cells), in order to achieve the required structure for a larger artery. The end result of arteriogenesis is that a pre-existent small artery grows into a large functional conductance artery. The initial stimulus for arteriogenesis is a change in the shear stress of the endothelial layer (not ischemia), which occurs through an increase in the blood flow through an artery.

It is therefore important to note that the present invention relates to arteriogenesis, the mechanism of which is fundamentally different from the mechanism of angiogenesis (Schaper 2009).

Previous studies have focused on the investigation of angiogenesis, whereby VEGF has been identified as a major regulator. Previous studies on rabbits, in which the femoral artery has been removed, show that under these hypoxic conditions VEGF is strongly up-regulated (Takeshita, Zheng et al. 1994). Additional application of the specific endothelial mitogen VEGF can stimulate artery growth. Such studies however do not distinguish between the outgrowth of capillaries (angiogenesis) and the mechanism related to the collateral growth of arteries (arteriogenesis). Angiogenesis occurs directly in the ischemic area and hypoxic conditions lead to the local production of VEGF. VEGF functions primarily as a mitogen for endothelial cells. However, arteriogenesis requires the reconstruction of endothelial cells, smooth muscles cells and a highly regulated end complex reconstruction of the extracellular matrix. In the case of an arterial obstructive or occlusive condition angiogenesis alone does not lead to a significant improvement in perfusion (Zadeh and Guha 2003, Storkebaum and Carmeliet 2004, Wang, Killic et al. 2005).

B1-receptor agonists are also known for modulating angiogenesis (Emaueli, Circulation, 2002), although only in relation to the outgrowth of capillaries (angiogenesis) and not in relation to modulation of the growth of collateral arteries (arteriogenesis).

In addition to flow-induced reconstruction, the migration of leucocytes plays a decisive role in arteriogenesis. Leucocytes, especially monocytes, produce inflammatory cytokines and growth factors, which are of significant importance for the remodelling of an artery. Such a remodelling leads to the enlargement of an arteriole into an artery with an outward growth of the artery lumen (positive outward remodelling). Arteriogenesis is therefore the most important endogenous compensatory mechanism for the maintenance of a stable blood flow in the case of a stenosis (mechanism of a biological bypass, Fig.1). Arteriogenesis however generally does not occur in the ischemic region but rather in regions proximal to the occlusion and therefore effects the bypass circulation which provides blood supply to the distal hypoperfusion area (Fig. 1) (Pipp, Heil et al. 2003).

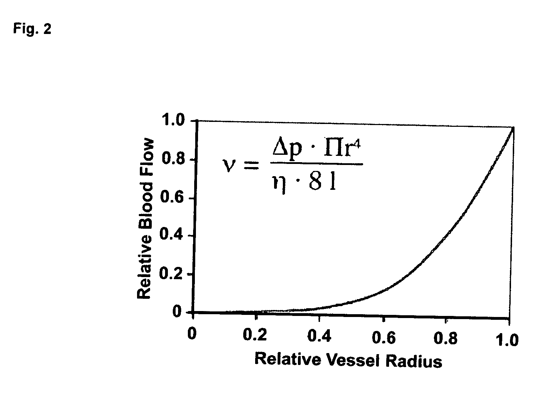

A schematic curve demonstrates the biological importance of arteriogenesis (Fig. 2). Angiogenesis produces many new capillaries with a small diameter where as arteriogenesis enlarges the diameter of already existing arteries. The Hagen-Poiseuille rule demonstrates the relationship between blood flow and artery diameter and shows that the blood flow is enhanced by an exponential factor of 4 in relationship to the radius (Fig. 2). According to this rule a minimal change in artery radius leads to significant improvements in perfusion. Comparable improvements in perfusion can not be achieved through the growth of many new small capillaries, as is the case in angiogenesis.

Previous studies have shown that collateral artery growth can be stimulated by an increase in blood flow (specifically an increase in the shear stress of the endothelial cells) or through the application of cytokines such as a GM-CSF (Buschmann, Busch et al. 2003). In a stroke or apoplexy model in the rat is has been shown that stimulation of arteriogenesis via the application of GM-CSF leads to an increase of the haemodynamic reserves of the brain (Schneeloch, Mies et al. 2004). Through the application of GM-CSF the ischemic region in an experimentally induced stroke could be significantly reduced. The stimulation of arteriogenesis is therefore an effective strategy for the treatment and/or prevention of ischemic obstructive or occlusive conditions, in addition to heart attacks and strokes.

Factors such as GM-CSF have been tested for their therapeutic stimulation of arteriogenesis (van Royen, Schirmer et al. 2005). The bradykinin signalling pathway has however not been examined in the prior art for its role in arteriogenesis, although its function as a vasodilator (antagonist of the Renin-angiotesin system) and its stimulatory effect in neo-vascularisation (Emanueli, Bonaria Salis et al. 2002) is known.

Bradykinin receptor 1 (B1 R) and bradykinin receptor 2 (B2R) belong to the kallikrein-kinin system (KKS), which also contains the proteins kallikrein and kininogen. Kininogen is a substrate of the KKS and is enzymatically cleaved by kallikrein, through which a number of kinins are released.

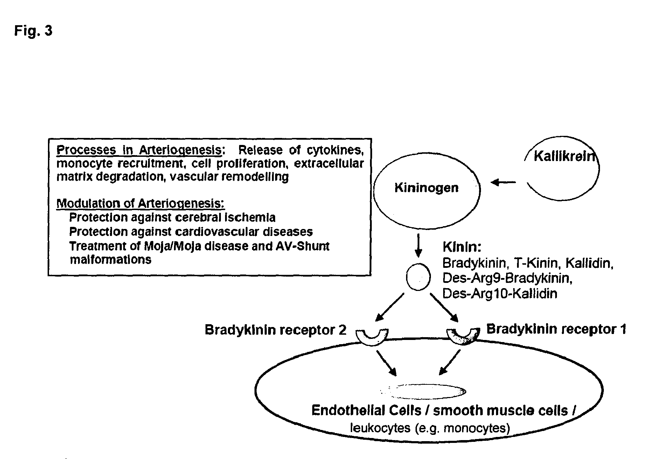

Bradykinin, T-kinin and kallidin all belong to the kinin group. These proteins can be digested at their C terminus by a carboxypeptidase and can therefore be transformed to Des-Arg⁹-bradykinin and Des-Arg ¹⁰ -kallidin. Bradykinin, T-kinin and kallidin impart their signalling effect via the B2-receptor and Des-Arg⁹-bradykinin and Des-Arg ¹⁰-kallidin via the B1-receptor (Fig. 3). All components of the kallikrein-kinin system can be produced in the artery wall. The bradykinin receptors in the artery walls can be expressed in either the endothelial cells or the smooth muscle cells. A signalling effect via the bradykinin-receptors leads to vasodilatation, cell proliferation, inflammation and the production of various cytokines.

The present invention therefore relates to a method for modulating collateral artery growth (arteriogenesis) through the inhibition or stimulation of the bradykinin receptor 1 (B1 R) and/or via the bradykinin receptor 2 (BR2) signalling pathways.

### SUMMARY OF THE INVENTION

In light of the prior art the technical problem underlying the present invention is to provide an effective method for modulating collateral vessel growth (arteriogenesis), whereby collateral artery growth can be either activated or inhibited, in addition to providing substances capable of such modulation and related medicaments.

This problem is solved by the features of the independent claims. Preferred embodiments of the present invention are provided by the dependent claims.

The invention therefore relates to a method for modulating collateral vessel growth (arteriogenesis) by the inhibition or stimulation of the bradykinin receptor 1 (B1 R) and/or the bradykinin receptor 2 (B2R) signalling pathways.

In one embodiment the method of the present invention is characterized in that the modulation of the collateral vessel growth occurs via the modulation of collateral arteries from existing arterial networks, preferably by modulation of arterial radius.

In one embodiment the method of the present invention is characterized in that the modulation, such as an induction or a reduction, of the collateral vessel growth occurs through the stimulation (using an agonist) or the inhibition (using an antagonist) of the bradykinin receptor 1 and/or bradykinin receptor 2 signalling pathways, preferably in the vessel wall by modulation of the bradykinin receptors of the endothelial cells, the smooth muscle cells and/or leucocytes that have migrated into the vessel wall.

In one embodiment the method of the present invention is characterized in that the induction of the collateral vessel growth (arteriogenesis) occurs by stimulation of the bradykinin receptor signalling pathways.

In a preferred embodiment the method of the present invention is characterized in that the bradykinin receptor agonist for induction of arteriogenesis is a nonapeptide bradykinin, structurally related kinin (for example T-kinin kallidin, R838, Des-Arg⁹-bradykinin or Des-Arg ¹⁰-kallidin), other structurally related molecule with modified amino acid sequence, a modified peptide and/or a non-peptide molecule.

In one embodiment the method of the present invention is characterized in that the inhibition of collateral vessel growth (arteriogenesis) occurs by inhibition of the bradykinin receptor signalling pathways.

In a preferred embodiment the method of the present invention is characterized in that the bradykinin receptor antagonist is a modified peptide such as icatibant (BR2 antagonist) or (Des-Arg⁹, Leu⁸)-bradykinin (BR1 antagonist), or other structurally related molecule with modified amino acid sequence, such as peptide, modified peptide or non-peptide molecules, which preferably bind to the bradykinin receptor, for example antibodies.

The invention further relates to a method as described above, characterized in that the modulation of the collateral vessel growth occurs by means of nucleic acid molecules, preferably through nucleic acids (such as siRNA or microRNA) with complementarity to mRNA of components of the kallikrein-kinin system (for example kininogen, BR1 and/or BR2).

The invention further relates to a method as described herein, characterized in that the bradykinin receptor pathway stimulation is enhanced by bradykinin augmentation as a consequence of ACE inhibitor activity.

A further aspect of the present invention relates to a bradykinin receptor 1 (B1 R) and/or bradykinin receptor 2 (B2R) modulating substance for use as a medicament to modulate collateral vessel growth (arteriogenesis) by the inhibition or stimulation of the bradykinin receptor 1 and/or the bradykinin receptor 2 signalling pathways.

A further aspect of the present invention relates to a bradykinin receptor 1 (B1 R) and/or bradykinin receptor 2 (B2R) inhibiting substance for use as a medicament to inhibit collateral vessel growth.

A further embodiment of the present invention relates to a bradykinin receptor 1 (B1 R) and/or bradykinin receptor 2 (B2R) stimulating substance for use as a medicament to stimulate collateral vessel growth.

A further aspect of the invention relates to a bradykinin receptor 1 (B1 R) and/or bradykinin receptor 2 (B2R) modulating substance for use as a medicament.

The bradykinin receptor 1 (B1 R) and/or bradykinin receptor 2 (B2R) stimulating substance of the present invention can be used as a medicament for the prevention and/or treatment of circulatory disorders, arterial conditions and/or ischemic obstructive or occlusive conditions or stroke, preferably coronary heart disease or peripheral obstructive disease, preferably via stimulation of the bradykinin signalling pathways.

A further aspect of the invention relates to a pharmaceutical composition comprising at least one bradykinin receptor 1 and/or bradykinin receptor 2 modulating substance with a pharmaceutically acceptable carrier.

The invention also relates to the use of a bradykinin receptor 1 (B1 R) and/or bradykinin receptor 2 (B2R) stimulating substance for the manufacture of a medicament to prevent and/or treat circulatory disorders, arterial diseases and/or ischemic obstructive or occlusive conditions or strokes, preferably coronary heart diseases or peripheral arterial obstructive diseases, preferably by a stimulation of the bradykinin signalling pathways.

A further aspect of the invention relates to a method for the therapeutic or prophylactic treatment of circulatory disorders, arterial diseases and/or ischemic obstructive or occlusive conditions or strokes, preferably coronary heart diseases or peripheral arterial obstructive diseases, characterised in that a therapeutically effective amount of a bradykinin receptor 1 (B1 R) and/or bradykinin receptor 2 (B2R) stimulating substance is administered to a mammalian patient.

### DETAILED DESCRIPTION OF THE INVENTION

The present invention relates to modulation of flow induced collateral vessel or artery growth and the outgrowth of collateral arterioles from pre-existing arterial networks. Further subject matter of the invention is the induction or reduction of collateral vessel growth through the stimulation (agonists) or inhibition (antagonist) of the bradykinin receptor 1 and/or bradykinin receptor 2 signalling pathways in the artery or blood vessel wall (modulation of the bradykinin receptors of the endothelial cells, the smooth muscle cells or the leucocytes that have migrated into the vessel or artery wall).

Collateral vessel growth or artery growth according to the present invention are terms than can be used to describe arteriogenesis. Arteriogenesis is characterised by the outgrowth of collateral arterioles from pre-existing arterial networks. The term "pre-existing arterial networks" can also be described as arterial networks with pre-existing arteriolar connections, such as pre-existing capillaries, arteries, vessels or other conduits of blood found in a mammalian organism. Therefore the present invention further relates to the use of a bradykinin receptor 1 (B1 R) and/or bradykinin receptor 2 (B2R) stimulating substance for modulating collateral growth of collateral arteries and/or other arteries from pre-existing arteriolar connections.

Agonists can be used for the induction of collateral vessel or artery growth (arteriogenesis) via the stimulation of the bradykinin receptor signalling pathways. Various BR-agonists can be used for the modulation of arteriogenesis, such as the nonapeptide bradykinin, structurally related kinins (T kinin, kallidin, Des-Arg⁹-bradykinin and Des-Arg ¹⁰-kallidin) in addition to further structurally related substitutes with modified amino acid sequences. The invention further relates to an induction of arteriogenesis through the stimulation of bradykinin receptors with non-peptide molecules.

The invention further relates to the inhibition of arteriogenesis via antagonists of the bradykinin receptors. As antagonists the following molecules are intended as examples: icatibant (BR2 antagonists) or (Des-Arg⁹, Leu⁸)-bradykinin (BR1 antagonists), in addition to related substitutes or alternatives with modified amino acid sequences. The inhibition of arteriogenesis via non peptide molecules, which preferably bind to the bradykinin receptor, is a further aspect of the invention. The invention also includes modulation of collateral blood vessel or artery growth by means of nucleic acid molecules, for example through nucleic acid molecules (siRNA, microRNA) with homology to the mRNA of components of the kallikrein-kinin systems (for example kininogen, BR1, BR2).

The present invention is relevant for the medical and pharmaceutical industries. A stimulation of arteriogenesis via the bradykinin receptor signalling pathways, particularly via a long-term application of a stimulant for the prevention of ischemic insults, is subject matter of the present invention. Further subject matter of this invention is therefore the prevention of strokes, coronary heart conditions or PAVKs via a stimulation of the bradykinin signalling pathways. Patients which have already exhibited the first warning signal of a stroke (the so called transitory ischemic attacks [TIA]), are one example of a patient group that could benefit from the therapeutic induction of arteriogenesis. Patients with such vessel or artery constriction could avoid the threatening stroke or coronary heart condition by an induction of a naturally occurring biological bypass via bradykinin receptor agonists, when applied at an early time point.

The present invention relates therefore to the prevention and/or therapy of circulatory disorders, cardiovascular disease, artery or blood vessel conditions and/or ischemic obstructive or occlusive diseases or conditions.

Circulatory disorders, cardiovascular disease, artery or blood vessel conditions and/or ischemic obstructive or occlusive diseases or conditions are terms that encompass a variety of diseases and conditions. The vascular diseases treated by the present invention are vascular diseases of mammals. The word mammal means any mammal. Some examples of mammals include, for example, pet animals, such as dogs and cats; farm animals, such as pigs, cattle, sheep, and goats; laboratory animals, such as mice and rats; primates, such as monkeys, apes, and chimpanzees; and humans. In some embodiments, humans are preferably treated by the methods of the invention.

The terms circulatory disorders, cardiovascular disease, artery or blood vessel conditions and/or ischemic obstructive or occlusive diseases or conditions refer to states of vascular tissue where blood flow is, or can become, impaired or altered from normal levels. Many pathological conditions can lead to vascular diseases that are associated with alterations in the normal vascular condition of the affected tissues and/or systems. Examples of vascular conditions or vascular diseases to which the methods of the invention apply are those in which the vasculature of the affected tissue or system is senescent or otherwise altered in some way such that blood flow to the tissue or system is reduced or in danger of being reduced or increased above normal levels. It refers to any disorder in any of the various parts of the cardiovascular system, which consists of the heart and all of the blood vessels found throughout the body. Diseases of the heart may include coronary artery disease, CHD, cardiomyopathy, valvular heart disease, pericardial disease, congenital heart disease (e.g., coarctation, atrial or ventricular septal defects), and heart failure. Diseases of the blood vessels may include arteriosclerosis, atherosclerosis, hypertension, stroke, vascular dementia, aneurysm, peripheral arterial disease, intermittent claudication, vasculitis, venous incompetence, venous thrombosis, varicose veins, and lymphedema.

Examples of vascular conditions that can be treated with the compositions and methods of the invention include atherosclerosis, preeclampsia, peripheral vascular disease, erectile dysfunction, cancers, renal failure, heart disease, and stroke. Vascular, circulatory or hypoxic conditions to which the methods of the invention apply also include those associated with, but not limited to, maternal hypoxia (e.g., placental hypoxia, preeclampsia), abnormal pregnancy, peripheral vascular disease (e.g., arteriosclerosis), transplant accelerated arteriosclerosis, deep vein thrombosis, erectile dysfunction, cancers, renal failure, stroke, heart disease, sleep apnea, hypoxia during sleep, female sexual dysfunction, fetal hypoxia, smoking, anemia, hypovolemia, vascular or circulatory conditions which increase risk of metastasis or tumor progression, hemorrhage, hypertension, diabetes, vasculopathologies, surgery (e.g., per-surgical hypoxia, post-operative hypoxia), Raynaud's disease, endothelial dysfunction, regional perfusion deficits (e.g., limb, gut, renal ischemia), myocardial infarction, stroke, thrombosis, frost bite, decubitus ulcers, asphyxiation, poisoning (e.g., carbon monoxide, heavy metal), altitude sickness, pulmonary hypertension, sudden infant death syndrome (SIDS), asthma, chronic obstructive pulmonary disease (COPD), congenital circulatory abnormalities (e.g., Tetralogy of Fallot) and Erythroblastosis (blue baby syndrome). In particular embodiments, the invention is a method of treating loss of circulation or endothelial dysfunction in an individual.

Thus, the invention is directed to compositions useful in a method of treating diseases such as stroke, atherosclerosis, acute coronary syndromes including unstable angina, thrombosis and myocardial infarction, plaque rupture, both primary and secondary (in-stent) restenosis in coronary or peripheral arteries, transplantation-induced sclerosis, peripheral limb disease, intermittent claudication and diabetic complications (including ischemic heart disease, peripheral artery disease, congestive heart failure, retinopathy, neuropathy and nephropathy), or thrombosis.

Circulatory disorders relate to various organs such as brain stem, heart, kidney, adrenal and lung. In addition, the present invention is useful for the prevention and treatment of the diseases induced by peripheral circulation failure in mammals, such as Raynaud's syndrome, arteriosclerosis obliterans, romsoongitis obliterans, Buerger disease, diabetic microvascular disorders and peripheral arterial obstruction (e.g. diabetic gangrene). Moreover, the present invention is useful for the prevention and treatment of hypertensive organ circulatory disorders, as well as for the treatment of cerebrovascular disorders both in acute stages and chronic stages.

Ischemic conditions include coronary arteriosclerosis, angina pectoris, and acute and old myocardial infarction and refers to the serious disorder of the vital heart that mostly affects males and females in late middle age or older. Coronary arteriosclerosis is characterized by arteriosclerosis in the coronary artery that supplies nutrients to the heart. Angina pectoris is characterized by attacks of chest pain caused by impaired blood flow in the coronary artery. Myocardial infarction is characterized by myocardial necrosis caused by impaired blood flow in the coronary artery and by fatal complications coming there with such as arrhythmia, cardiac failure, cardiac rupture, and pump failure. Impaired blood flow to the heart, a vital organ, is an essential characteristic of these ischemic heart diseases

Peripheral arterial occlusive disease (PAOD; also referred to as peripheral arterial disease (PAD)) results either from atherosclerotic or inflammatory processes producing arterial stenosis, or from thrombus formation associated with underlying atherosclerotic disease. A common site for PAOD is in the lower limbs. This process of atherosclerosis causes initial thickening and plaque formation encroaching the arterial lumen, decreasing the effective luminal radius of afflicted arterial segments, producing an anatomic and sometimes functional obstruction to blood flow.

It is important to note that a stimulation of angiogenesis (neovascularisation) or a vasodilatation does not lead to a preventative treatment for patients with progressive constriction of blood vessels or arteries (for example as it is the case in atherosclerosis). A stimulation of angiogenesis can not sufficiently compensate for an endogenous stenosis in a major artery (see above prior art comparing angiogenesis and arteriogenesis). Similarly dilatation to its maximum extent cannot compensate for an endogenous stenosis in a major artery, as the hemodynamic reserves become very quickly exhausted when collateral vessels or arteries do not sufficiently grow in their diameter through arteriogenesis.

Conditions such as the Moja Moja syndrome can however be treated through modulation of arteriogenesis. The Moja Moja condition is characterized by closing of the internal carotid artery, whereby the blood flow can however be maintained over collateral arteries. A stimulation of the collateral blood flow can in such cases lead to an enhanced perfusion in a hypoperfusion area.

Flow induced collateral blood vessel growth has not only a physiological importance but can also lead to a pathological formation of collateral vessels and arteries, especially in cases of etiologically unclear brain diseases involving the arterio-venous vessel malformation. An AV-shunt can usually be treated using a medical gel in an operative situation in order to close the shunt. This treatment of arterio-venous malformation can however lead to the induction of collateral blood vessels and arteries, which after the operation can however lead to the further growth of collateral arteries and therefore formation of a new shunt. In such cases the inhibition of arteriogenesis through the bradykinin receptor antagonists (for example as part of an AV-shunt gel) would be useful.

As discussed above, the active substances of the invention may be used in a pharmaceutical composition or medicament when combined with a pharmaceutically acceptable carrier. The term "pharmaceutically acceptable" means a non-toxic material that does not interfere with the effectiveness of the biological activity of the active ingredient(s). The characteristics of the carrier will depend on the route of administration. Such a composition may contain, in addition to the active substance and carrier, diluents, fillers, salts, buffers, stabilizers, solubilizers, and other materials well known in the art. Formulation of pharmaceutically-acceptable excipients and carrier Solutions is well-known to those of skill in the art, as is the development of suitable dosing and treatment regimens for using the particular compositions described herein in a variety of treatment regimens, including e.g., oral, parenteral, intravenous, intranasal, and intra-muscular administration and formulation.

The medicament, otherwise known as a pharmaceutical composition, containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated.

The present invention refers to a pharmaceutical composition for topical application, oral ingestion, inhalation, or cutaneous, subcutaneous, or intravenous injection. Topical application is one preferred embodiment, especially via a cream or gel. Topical application forms relate to gels, creams, suppositories, or films. The pharmaceutical composition of the invention may be in the form of a liposome in which the active substance of the invention is combined, in addition to other pharmaceutically acceptable carriers, with amphipathic agents such as lipids which exist in aggregated form as micelles, insoluble monolayers, liquid crystals, or lamellar layers which are in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art.

When a therapeutically effective amount of the active substance of the invention is administered by intravenous, cutaneous or subcutaneous injection, the active substance will be in the form of a pyrogen-free, parenterally acceptable aqueous solution. The preparation of such parenterally acceptable solutions, having due regard to pH, isotonicity, stability, and the like, is within the skill in the art. A preferred pharmaceutical composition for intravenous, cutaneous, or subcutaneous injection should contain, in addition to the active subtance, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

As used herein, the term "therapeutically effective amount" means the total amount of each active component of the pharmaceutical composition or method that is sufficient to show a meaningful patient benefit.

The amount of active substance in the pharmaceutical composition of the present invention will depend upon the nature and severity of the condition being treated, and on the nature of prior treatments which the patient has undergone. Larger doses may be administered until the optimal therapeutic effect is obtained for the patient, and at that point the dosage is not increased further.

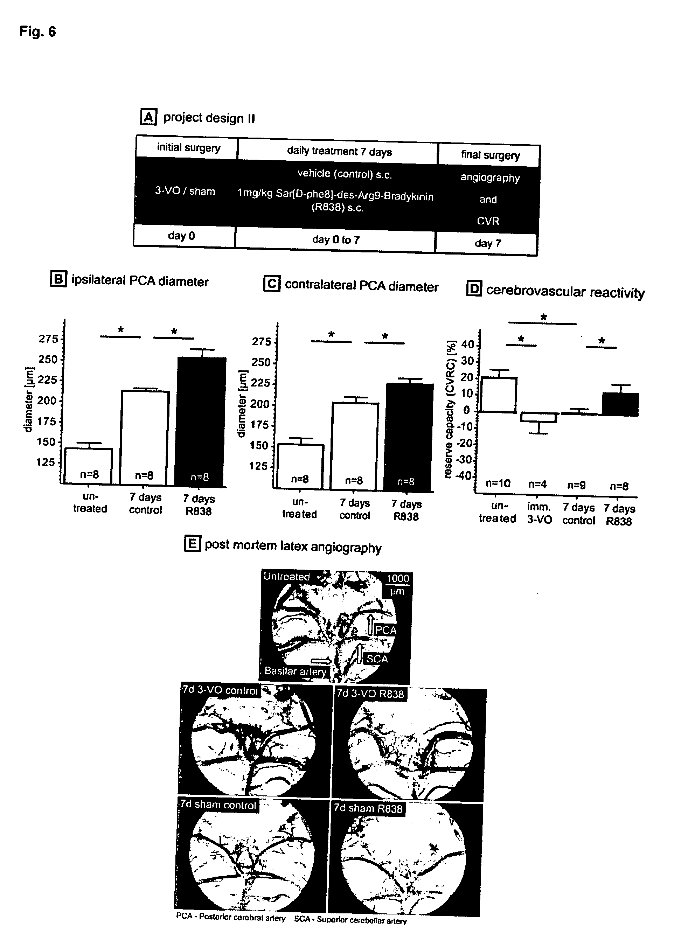

Ligand B1 and B2 receptor agonism plays beneficial and protecting roles in cardiovascular-related pathologies such as ischaemic heart diseases, periphery artery diseases and stroke. The present invention indicates that a strong cardioprotective effect is provided by bradykinin receptor signalling to therapeutically enhance arteriogenesis. Therefore it is suggested to therapeutically stimulate arteriogenesis via the B2 receptors and preferably via the B1 receptor pathway (see Figure 6). Stimulation of collateral artery growth in case of obstruction or occlusion of a major artery, enhancing blood flow into the hypoperfused area distal of the occlusion, can prevent ischemia.

Interestingly, both bradykinin receptors exhibit different biological features, such as that B2 receptors are considered to be constitutively expressed and ready to respond to newly formed kinins. Upon ligand binding B2 receptors are rapidly desensitized and degraded. In contrast, Bradykinin receptor 1 is expressed upon induction. B1 receptors generally exhibit long term effects. Since signalling via B1 or B2 receptors will result in different signalling pathways, stimulation of bradykinin receptor 2 or bradykinin receptor 1 by selective compound selection can enable distinct options in therapeutic-induced collateral growth. Here, B1 or B2 receptors show distinct affinity for different bradykinin compounds. Naturally, Bradykinin B2 receptors have high affinity for bradykinin and kallidin (lys-bradykinin). In contrast, the Bradykinin receptor 1 exhibits a high affinity for des-Arg9-bradykinin and des-Arg9-kallidin. These natural bradykinin receptor ligands are subjected to rapid inactivation by tissue and plasma enzymes, which limit their stability. However, many bradykinin compounds have been designed with improved properties for clinical intervention. For example, stimulation by orally active and selective bradykinin 2 receptor ligands for clinical intervention in different disease models was tested for the non-peptide compounds FR-190997 and FR-191413 (Fujisawa). Such studies might encourage the required efforts to identify more suited candidates.

The best-characterized ligand for the Bradykinin receptor 1 signalling peptide, Sar[DPhe8]desArg9BK, exhibits a lower susceptibility toward proteolysis and a moderate affinity and potency (about 11- and 2-fold less, respectively) relative to the natural ligand Lys-desArg-bradykinin (Cote, 2009). Figure 6 shows how cerebral arteriogenesis can be therapeutically enhanced by stimulation with Sar[DPhe8]desArg-bradykinin. Here it is demonstrated that B1 R agonists bearing optimized pharmacological features (high potency, selectivity and stability toward proteolysis) hold promise as valuable therapeutic agents in the treatment of ischemic diseases.

Tables 1 and 2 provide a list of bradykinin receptor ligands for use in modulating (inhibit/activate) arteriogenesis. Table 3 provides a list of publications, which are incorporated by reference in their entireties, in which bradykinin receptor modulators are disclosed that can be used for modulating arteriogenesis via bradykinin receptor signalling.

**Table 1. List of bradykinin receptor signalling agonists that can be used to modulate arteriogenesis.**

| **Agonist** | **B-Receptor** | **Substance** |
|---|---|---|
| **Bradykinin/H-1970** | B2 | Peptide |
| natural peptide | | |
| **Des-Arg9-Bradykinin** | B1 | Peptide |
| natural peptide | | |
| **Kallidin/Lys-Bradykinin/H-2180** | B2 | Peptide |
| natural peptide | | |
| **(Des-Arg10)-Kallidinl Lys-(Des-Arg9)-Bradykinin** | B1 | Peptide |
| natural peptide | | |
| **Sar-[D-Phe8]-des-Arg9-Bradykinin** | B1 | Modified Peptide |
| potent, selective, resistant to degradation | | |
| **SarLys[Hyp3, Cha5, DPhe8]desArg9BK** | B1 | Modified Peptide |
| potent, selective, resistant to degradation | | |
| **SarLys[Hyp3, lgl5, DPhe8]desArg9BK** | B1 | Modified Peptide |
| potent, selective, resistant to degradation | | |
| **SarLys[Hyp3, Cpg5, DPhe8]desArg9BK** | B1 | Modified Peptide |
| potent, selective, resistant to degradation | | |
| **RMP-7 lobradamil / Cereport** | B2 | Modified Peptide |
| potent, selective, resistant to degradation | | |
| **JMV-1116** | B2 | Modified Peptide |
| potent, selective, resistant to degradation | | |
| **lie-Ser-BK (B 1643)** | B2 | Modified Peptide |
| | | |
| [**Phe8-(ψ-CH2NH)-Arg9]-BK** | B2 | Modified Peptide |
| | | |
| **Hyp3,Tyr(Me)8]-BK** | B2 | Modified Peptide |
| | | |
| **JMV1116 and analogues** | B2 | Modified Peptide |
| | | |
| **[Hyp3]-BK (B 7775)** | 82 | Modified Peptide |
| | | |
| FR-190997 | B2 | Non-Peptide |
| 4-(2-pyridylmethoxy)-substituted quinoline | | |
| FR-191413 | B2 | Non-Peptide |
| 3-(2-pyridylmethyl)-substituted benzimidazole | | |

**Table 2. List of bradykinin receptor signalling antagonists that can be used to modulate arteriogenesis.**

| **Antagonist** | **B-Receptor** | **Substance** |
|---|---|---|
| **AC-Lys-Arg-Pro-Pro-Gly-Phe-Ser-DβNal-lie** | B1 | Modified Peptide |
| **R 715** | | |
| **AC-Lys-Arg-Pro-Pro-Gly-(αMe)Phe-Ser-DβN al-lie** | B1 | Modified Peptide |
| **R892** | | |
| **(Des-Arg9,Leu8)-Bradykinin** | B1 | Peptide |
| | | |
| **[Leu8,des-Arg9]-BK** | 81 | Modified Peptide |
| | | |
| **[Leu9,des-ArglO]-Kallidin (CP0298)** | B1 | Modified Peptide |
| | | |
| **[des-Arg10]-HOE140** | B1 | Modified Peptide |
| | | |
| **Lys-[Leu8,desArg9]-BK** | B1 | Modified Peptide |
| | | |
| **(B9858: H-Lys-Lys-Arg-Pro-** | B1 | Modified Peptide |
| **Hyp-G ly-lgl-Ser-D-lgl-Oic-OH)** | | |
| **B-9430 (H-D-Arg-Arg-Pro-Hyp-Gly-Igl-Ser-** | B2 + B1 | Modified Peptide |
| **D-lgl-Oic-Arg-OH)** | | |
| **Icatibant/Hoe140/H157** | B2 | Modified Peptide |
| **FIRAZYR** | | |
| FR-167344 | B2 | Non-Peptide |
| | | |
| FR-173657 | B2 | Non-Peptide |
| | | |
| **D-Arg[Hyp3,Thi5,HypE(trans-propyl)7,Oic8]-BK** | B2 | Modified Peptide |
| NPC17731 | | |
| **WIN 64338** | B2 | Non-Peptide |
| | | |
| **Bradyzide** | B2 | Non-Peptide |
| | | |

**Table 3. List of bradykinin receptor modulators that can be used in the in the present invention.**

| Inventor | Patent;year | B receptor Antagonist |
|---|---|---|
| Boehringer Ingelheim Int | W02006048209; 2006 | B1R |
| Boehringer Ingelheim Int | W02008022945; 2008 | B1R |
| Boehringer Ingelheim Int | W02009013299; 2009 | B1R |
| Elan Pharmaceutical, Inc | W02006071775; 2006 | B1R |
| Elan Pharmaceutical, Inc | W02006113140; 2006 | B1R |
| Evotec Neurosciences GMBH | W02008125570; 2008 | B1R |
| Richter Gedeon Nyrt | W02007072092; 2007 | B1R |
| Richter Gedeon Nyrt | W02008050167; 2008 | B1R |
| Richter Gedeon Nyrt | W02008050168; 2008 | B1R |
| Richter Gedeon Nvrt | WO2008068540: 2008 | B1R |
| Grünenthal GMBH | W02008040492; 2008 | B1R |
| Grünenthal GMBH | W02008046573; 2008 | B1R |
| Grünenthal GMBH | WO2008131946; 2008 | B1R |
| Jerini AG | W02007003411; 2007 | both |
| Jerini AG | W02008116620; 2008 | B2R |
| Menarini | W02006040004; 2006 | B2R |
| Menarini | W02003103671; 2003 | B2R |
| Merck & Co | US20050261327;2005 | B1R |
| Merck & Co | US20060122236; 2006 | B1R |
| Merck & Co | US20060173023; 2006 | B1R |
| Merck & Co | W02005016886; 2005 | B1R |
| Merck & Co | US20060106011; 2006 | B1R |
| Merck & Co | US20060111392; 2006 | B1R |
| Merck & Co | US20060128765; 2006 | B1R |
| Merck & Co | W02005004810; 2005 | B1R |
| Neurogen Corporation | W0200701007; 2007 | B1R |
| Neurogen Corporation | WO2007140383; 2007 | B1R |
| Neurogen Corporation | W02008002849; 2008 | B1R |
| Neurogen Corporation | W02008024692; 2008 | B1R |
| Neurogen Corporation | W02008033739; 2008 | B1R |
| Sherbrooke University | W02006017938; 2006 | B1R |
| Sherbrooke University | US7211566; 2007 | B1R |
| Park Choo | W02007108566; 2007 | B1R |
| Park Choo | WO2007142431; 2007 | B1R |
| University of Colorado | W02008124756; 2008 | B1R |

Bradykinin is rapidly hydrolysed and degraded by ACE. Locally increased kinin concentrations, arising as a consequence of the blockade of ACE as the major kinin-degrading enzyme, significantly contribute to the apparent beneficial effects of ACE inhibitors, including their infarct size-reducing effects. In addition to direct Bradykinin receptor agonists and antagonists, Table 4 provides a comprehensive list of angiotensin converting enzyme (ACE) inhibitors.

**Table 4. Sample list of ACE-Inhibitors for enhancing modulation of arteriogenesis via bradykinin receptor signalling**

| **ACE-Inhibitors** | Obtained from |
|---|---|
| **Ramipril** | Roche, AwD-Pharma, Schwarz-Pharma/Sanol, R.A.N.-Pharm, ratiopharm, TAD Pharma, betapharm , Hennig, Abott, CT Arzneimittel, Wörwag, HEXAL, Winthrop, 1 A Pharma, AbZ-Pharma, ALIUD PHARMA, Sandoz, STADApharm, Schwarz Pharma |
| | |
| **Quinapril** | |
| | |
| **Cilazapril** | |
| | |
| **Spirapril-HCI 1H20** | |
| | |
| **Catopril** | |
| | |
| **Enalaprilmaleat** | |
| | |
| **Lisinopril** | |
| | |
| **Bencapril-HCI** | |
| | |
| **Perindopril-Erbumin** | |
| | |
| **Trandolapril** | |
| | |
| **Fosinopril-Natrium** | |

### FIGURES

- Figure 1:: Schematic of the flow induced collateral arterial system (arteriogenesis).
- Figure 2:: Relationship between blood flow and artery radius (The Law of Hagen-Poiseuille).
- Figure 3:: Schematic representation of the kallikrein-kinin system.
- Figure 4:: Measurement of collateral perfusion in the femoral ligature model in the mouse
- Figure 5:: Pharmacological inhibition of bradykinin receptor 1 and 2 signalling in the 3-vessel occlusion (3-VO) cerebral arteriogenesis model in rat.
- Figure 6:: Therapeutic stimulation of arteriogenesis with a selective BR1 agonist in the 3-vessel occlusion (3-VO) cerebral arteriogenesis model in rat.

### DETAILED DESCRIPTION OF THE FIGURES

**Figure 1****: Schematic of the flow induced collateral arterial system (arteriogenesis).**
   An artery occlusion of a major vessel leads to redistribution of blood flow by the recruitment of pre existing collateral vessels. Upon enhanced blood flow small arteries are remodelled into functional conductance arteries to supply hypoperfusion area with blood (Arteriogenesis - biological bypass).
**Figure 2****: Relationship between blood flow and artery radius (The Law of Hagen-Poiseuille).**
   Equation clarifies the relationship between flow and vessel radius to the power of 4. Flow volume v, viscosity n, pressure differences AP, distance differences (vessel length) Δl, radius r. A small increase in vessel radius leads to a significant enhancement of blood flow.
**Figure 3****: Schematic representation of the kallikrein-kinin system.**
   Kininogen is processed by kallikrein to cleave bradykinin, kallidin and other kinins. Kinins signal via the constitutively expressed B2 receptor and the inducible B1 receptor. Stimulation of bradykinin receptors may activate intracellular signalling pathways in endothelial cells, smooth muscle cells and leucocytes, such as monocytes. This results in cell proliferation, cell migration and leucocyte recruitment, which are features of arteriogenesis. Collateral growth can be modulated by stimulation/inhibition of the bradykinin receptor signalling.

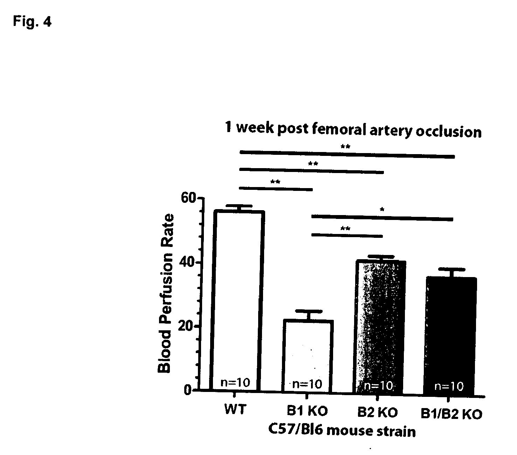

**Figure 4****: Measurement of collateral perfusion in the femoral ligature model in the mouse (7 days post femoral artery ligation)**
   Arteriogenesis was measured in bradykinin receptor 1 knock out (KO) mice, bradykinin receptor 2 KO mice, bradykinin receptor 1 and 2 double KO mice and in wild type (WT) mice. All bradykinin receptor knock out mice exhibit a markedly reduced arteriogenesis. The B1 KO mice have the strongest phenotype and arteriogenesis is severely reduced in comparison with WT mice (more than 50% reduction in blood perfusion rate).

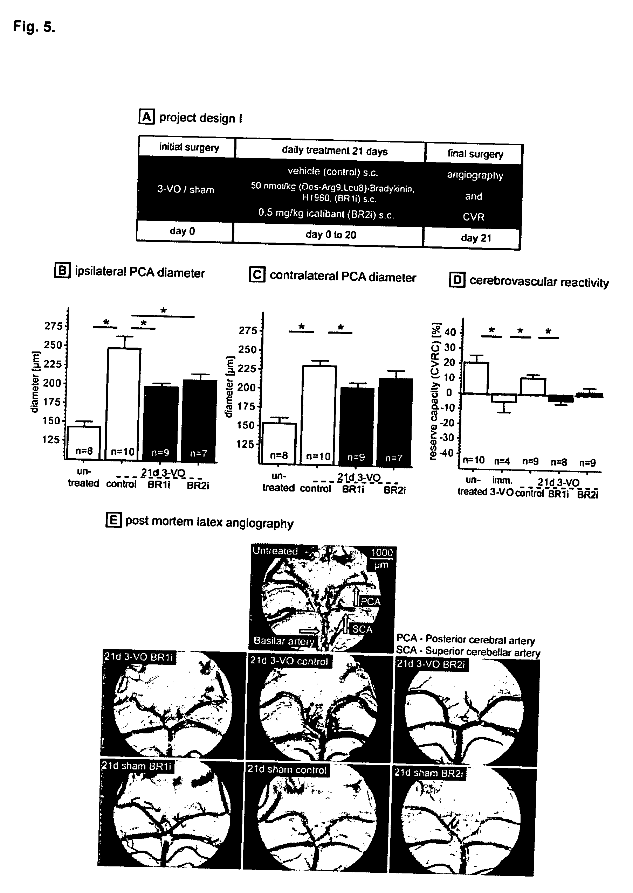

**Figure 5****: Pharmacological inhibition of bradykinin receptor 1 and 2 signalling in the 3-vessel occlusion (3-VO) cerebral arteriogenesis model in rat.**
   A. project design I. H-1960 is used as bradykinin receptor 1 (BR1) antagonist, Icatibant is used as BR2 antagonist. B,C. Diameters of the collateral pathway (posterior cerebral artery) ipsilateral (B) and contralateral (B) of the carotid artery ligation. D. Cerebrovascular reactivity. Collateral diameters (B,C) and cerebrovascular reactivities (D) are given for untreated animals (without 3-VO surgery), for control animals (3 weeks post 3-VO surgery without plus vehicle), for BR1 inhibition (3 weeks post 3-VO plus bradykinin receptor 1 antagonist treatment) and for BR2 inhibition (3 weeks post 3-VO bradykinin receptor 2 antagonist treatment). E. Brain vessel angiographies of untreated animals, post 3-VO and sham controls
**Figure 6****: Therapeutic stimulation of arteriogenesis with a selective BR1 agonist in the 3-vessel occlusion (3-VO) cerebral arteriogenesis model in rat.**
   A. project design II. R838 is used as bradykinin receptor 1 (BR1) agonist. B,C. Diameters of the collateral pathway (posterior cerebral artery) ipsilateral (B) and contralateral (B) of the carotid artery ligation. B,C. Diameters of the collateral pathway (posterior cerebral artery) ipsilateral (B) and contralateral (B) of the carotid artery ligation. D. Cerebrovascular reactivity. Collateral diameters (B,C) and cerebrovascular reactivities (D) are given for untreated animals (without 3-VO surgery), for control animals (1 weeks post 3-VO surgery plus vehicle), and for BR1 stimulation (1 weeks post 3-VO plus R838 treatment) E. Brain vessel angiographies of untreated animals, post 3-VO and sham controls.

### EXAMPLES

### Example 1: Measurement of collateral perfusion in the femoral ligature model in the mouse

Arteriogenesis was examined in an arteriogenesis model (ligation of the femoral artery) on the hind leg of C57/B16 mice. The ligature of the femoral artery in hind leg of a mouse is an established model for examining arteriogenesis (Hoefer, van Royen et al. 2001). Here arteriogenesis is measured through the collateral perfusion rate of the hind leg arteries by pressure controlled perfusion with fluorescent microspheres (Molecular Probes) of various sizes (Bergmann, Hoefer et al. 2006). In brief, a number of fluorescent microspheres where normalized against a reference number of blue microspheres. Values are expressed as percent perfusion by comparing the ligated vs. the unligated leg. Calculating collateral perfusion rate is a functional measurement to evaluate collateral growth (arteriogenesis). Finally arteriogenesis can be estimated by a count of microspheres using flow cytometry (MACS Quant, Miltenyi). Of all the functional measurements, the measurement of collateral growth by microsphere perfusion is considered to be the gold standard in evaluating arteriogenesis (Schaper 2009).

Collateral blood vessel growth is inhibited in BR1, BR2 and BR1/BR2 double -receptor knock out (KO) mice in comparison to wild type mice (WT) (Figure 4). Arteriogenesis is significantly inhibited in BR2-receptor knock out and particular, bradykinin receptor BR1 KO mice (22.41 units) exhibit a more than 50 % reduced collateral perfusion rate as compared to WT mice (56.17 units). In conclusion it was shown that the bradykinin receptor signalling pathway has a significant relevance for the modulation of arteriogenesis (molecular biological demonstration).

### Example 2: Pharmacological inhibition of bradykinin receptor 1 and 2 signalling in the 3-vessel occlusion (3-VO) cerebral arteriogenesis model in rat.

Pharmacological modulation of arteriogenesis was analysed using a 3 vessel occlusion (3-VO) model for cerebral arteriogenesis in the rat (Occlusion of both vertebral arteries and one carotid artery). Upon occlusion of three of four blood-supplying arteries to the brain, blood flow is redistributed by the recruitment of collateral pathways such as the posterior cerebral artery to supply the hypoperfusion area with blood. The 3-VO model in the rat has been established since 2003 and is acknowledged as a hypoperfusion model for testing cerebral arteriogenesis (Busch, Buschmann et al. 2003). By using the 3-VO model, relevant therapeutic stimulation of arteriogenesis was shown for instance by the application of GM-CSF (Buschmann, Circulation 2003).

Either by inhibition or stimulation of the bradykinin receptor signalling pathways, arteriogenesis was determined and physiological parameters of the brain were tested. Here, pharmacological modulation of collateral growth in the 3-VO model demonstrate that arteriogenesis can be inhibited through the selective B1 antagonists (Des-Arg⁹, Leu⁸)-bradykinin (H1960) and the selective B2-receptor antagonist HOE-140 (Icatibant) (Figure 5.). It is additionally demonstrated that cerebral arteriogenesis can be stimulated the selective BR1 agonist Sar- [D-Phe]des -Arg⁹-bradykinin (R838) (Figure 6.).

The characterisation of arteriogenesis was carried out via measurement of the artery or blood vessel diameter (posterior cerebral artery - PCA) in addition to via a measurement of the cerebrovascular reactivity (perfusion reserves in the brain). Cerebrovascular anatomy was studied after maximal vasodilation by a modification of the postmortem latex perfusion method of Maeda et al (Maeda, Hata et al. 1998) . Through a measurement of the cerebrovascular reactivity the CO2 partial pressure in the blood was artificially increased (by means of a carboanhydrase-inhibitor acetazolamide) and the reaction of the artery was measured via a measurement of blood flow via laser-doppler cytometry.

Figure 5B and 5C demonstrate that collateral arterial growth is strongly reduced in response to the selective inhibition of the bradykinin receptor 1 signalling pathway and slightly in response to the selective inhibition of the bradykinin receptor 2 signalling. In the case of previous treatment with a bradykinin receptor B1 antagonist, it is demonstrated that the arteries increase in diameter to a lesser extent than in the untreated controls. Here collateral growth is reduced in both collateral pathways (ipsilateral and contralateral of the occluded carotide artery). Experiments which examine the diameter changes after application of a bradykinin receptor B2 antagonist demonstrate reduced collateral growth.

CVR is reduced through selective inhibition of the bradykinin B1 and B2 signalling pathways. Negative CVR values are based on the Steal phenomenon (Kuwabara, lchiya et al. 1995). (Figure 5D).Upon increase of the CO2 partial preassure under normal conditions, vessels in the brain are capable of increasing blood flow to around 20% of the normal blood flow . Directly after the 3-VO experiment no cerebrovascular reactivity is detectable anymore. Blood flow recovers within 3 weeks post 3-VO and the perfusion reserve is restored to around 10%. In contrast, three weeks after 3-VO, no increase in blood flow is detected in animals treated with bradykinin receptor inhibitors (BR1 i, BR2i); thereby demonstrating that arteriogenesis has been effectively inhibited.

### Example 3: Therapeutic stimulation of arteriogenesis with a selective BR1 agonist in the 3-vessel occlusion (3-VO) cerebral arteriogenesis model in rat.

Figure 6 demonstrates therapeutic stimulation of arteriogenesis by using a selective bradykinin receptor 1 agonist Sar-[D-Phe]des-Arg⁹-bradykinin (R838). In the case of simultaneous application of the bradykinin receptor 1 agonist R838, it is demonstrated that seven days after 3-VO the diameter of the posterior cerebral artery (PCA) is significantly greater than the diameter in untreated rats after 3-VO (Figure 6B,C). Furthermore figure 6D. shows that CVR is significantly increased through stimulation with a selective bradykinin receptor 1 agonist R838 (Sar-[D-Phe]des-Arg⁹-bradykinin). Arteriogenesis can by stimulated therapeutically by application of R838 for 1 week.

In summary the examples demonstrate that cerebral arteriogenesis is significantly inhibited by antagonists via blocking the bradykinin receptors (Figure 5). Stimulation with a selective B1R agonist demonstrates that the cerebral arteriogenesis can also be stimulated (Figure. 6).

Rats have been used in the present examples as a model for mammals. The present invention and method according to the present invention are therefore also applicable for application or the treatment of all mammals, especially humans.

## Claims

1. Method for modulating collateral vessel growth (arteriogenesis) by the inhibition or stimulation of the bradykinin receptor 1 (B1 R) and/or the bradykinin receptor 2 (B2R) signalling pathways.

2. Method according to the preceding claim, **characterized in that** the modulation of the collateral vessel growth occurs via the modulation of collateral arteries from existing arterial networks, preferably by modulation of arterial radius.

3. Method according to one of the preceding claims, **characterized in that** the modulation, such as an induction or a reduction, of the collateral vessel growth occurs through the stimulation (using an agonist) or the inhibition (using an antagonist) of the bradykinin receptor 1 and/or bradykinin receptor 2 signalling pathways, preferably in the vessel wall by modulation of the bradykinin receptors of the endothelial cells, the smooth muscle cells and/or leucocytes that have migrated into the vessel wall.

4. Method according to one of the preceding claims, **characterized in that** the induction of the collateral vessel growth (arteriogenesis) occurs by stimulation of the bradykinin receptor signalling pathways.

5. Method according to the preceding claim, **characterized in that** the bradykinin receptor agonist for induction of arteriogenesis is a nonapeptide bradykinin, structurally related kinin (for example T-kinin kallidin, R838, Des-Arg⁹-bradykinin or Des-Arg¹⁰-kallidin), other structurally related molecule with modified amino acid sequence, a modified peptide and/or a non-peptide molecule

6. Method according to one of the preceding claims, **characterized in that** the inhibition of collateral vessel growth (arteriogenesis) occurs by inhibition of the bradykinin receptor signalling pathways.

7. Method according to the preceding claim, **characterized in that** the bradykinin receptor antagonist is a modified peptide such as icatibant (BR2 antagonist) or (Des-Arg⁹, Leu⁸)-bradykinin (BR1 antagonist), or other structurally related molecule with modified amino acid sequence, such as peptide, modified peptide or non-peptide molecules, which preferably bind to the bradykinin receptor, for example antibodies.

8. Method according to one of the preceding claims, **characterized in that** the modulation of the collateral vessel growth occurs by means of nucleic acid molecules, preferably through nucleic acids (such as siRNA or microRNA) with complementarity to mRNA of components of the kallikrein-kinin system (for example kininogen, BR1 and/or BR2).

9. Method according to one of the preceding claims, **characterized in that** the bradykinin receptor pathway stimulation is enhanced by bradykinin augmentation as a consequence of ACE inhibitor activity.

10. Bradykinin receptor 1 (B1 R) and/or bradykinin receptor 2 (B2R) modulating substance for use as a medicament to modulate collateral vessel growth (arteriogenesis) by the inhibition or stimulation of the bradykinin receptor 1 and/or the bradykinin receptor 2 signalling pathways.

11. Bradykinin receptor 1 (B1 R) and/or bradykinin receptor 2 (B2R) inhibiting substance for use as a medicament to inhibit collateral vessel growth.

12. Bradykinin receptor 1 (B1 R) and/or bradykinin receptor 2 (B2R) stimulating substance for use as a medicament to stimulate collateral vessel growth.

13. Bradykinin receptor 1 (B1 R) and/or bradykinin receptor 2 (B2R) stimulating substance according to the previous claim for use as a medicament for the prevention and/or treatment of circulatory disorders, arterial conditions and/or ischemic obstructive or occlusive conditions or stroke, preferably coronary heart disease or peripheral obstructive disease, preferably via stimulation of the bradykinin signalling pathways.

14. Bradykinin receptor 1 (B1 R) and/or bradykinin receptor 2 (B2R) modulating substance for use as a medicament.

15. Pharmaceutical composition comprising at least one bradykinin receptor 1 and/or bradykinin receptor 2 modulating substance with a pharmaceutically acceptable carrier.
